# EUROPEAN PATENT APPLICATION

(11) **EP 4 155 732 A1**
(43) Date of publication of application: **29.03.2023**
(21) Application number: 21809725.1
(22) Date of filing: 11.05.2021
(51) Int. Cl.: G01N 33/36, D06H 3/02, A61L 2/00, D06M 13/10, D06M 13/282, G01N 31/22

(54) **BIOINDICATOR FOR USE IN VALIDATING THE EFFECTIVENESS OF THERMAL DISINFECTION PROCESSES AGAINST CORONAVIRUSES AND VALIDATION METHOD**

(30) Priority: 20.05.2020 ES 202030465
(71) Applicant: Asociación de Investigación de la Industria Textil (AITEX), 03801 Alcoy (Alicante) (ES)
(72) Inventor: CAMBRA SÁNCHEZ, Vicente, 03801 Alcoy (Alicante) (ES); BLANES COMPANY, María, 03801 Alcoy (Alicante) (ES); SANJOSÉ JOVER, Juan, 03801 Alcoy (Alicante) (ES); BAS VALOR, Blanca, 03801 Alcoy (Alicante) (ES); SOLBES GISBERT, Lorena, 03801 Alcoy (Alicante) (ES)
(74) Representative: Ibarra Garcia, Isabel
(86) International application number: PCT/ES2021/070327
(87) International publication number: WO 2021/234198

(57) **Abstract**

The invention relates to a biomarker formed by a suspension of lipid particles formed by vesicles with an outer membrane of phospholipids the hydrophobic tail of which is oriented towards the inside of the vesicle such that the outer membrane encloses a fat-soluble coloring agent which will be released when the rupturing of the mentioned outer membrane has been caused. In this way, a textile material is impregnated with the biomarker acting as a marker or indicator simulating the behavior of coronavirus, for use thereof in the validation of the effectiveness of disinfection processes for clothing and different textile substrates.

## Description

### OBJECT OF THE INVENTION

The present invention relates to a biomarker consisting of a suspension of lipid particles containing a fat-soluble coloring agent, such that a preferably white textile material is impregnated with the suspension, enabling the human eye to identify whether the rupturing of the outer membrane or envelope of the mentioned lipid particles has taken place after a specific thermal disinfection treatment.

The developed biomarker, as a result of its size and morphology, exhibits a behavior similar to coronavirus, so it allows confirming that the textile material impregnated with the suspension of the invention has been subjected to certain disinfection treatment for a specific exposure time which is sufficient to ensure that possible particles of the mentioned coronavirus present in said clothing have become inactive.

### BACKGROUND OF THE INVENTION

Coronaviruses (CoV) are particles with a size of 120 nm to 160 nm and a viral envelope or outer membrane. The outer membrane consists of a lipid bilayer in which at least three protein structures are embedded: a membrane protein, a second protein the main function of which is viral assembly, and a third protein constituting spikes or protrusions emerging from the outer membrane and allowing the virus to penetrate the host cells under attack.

Additionally, CoV particles include RNA (ribonucleic acid) therein.

The mentioned spikes form protrusions on the surface of the virus which end in a wider crest, giving it a shape reminiscent of a crown, which is the origin of the name of the microorganism.

The presence of coronavirus is causing serious diseases in humans, leading to a worldwide pandemic which has changed human habits.

At present, the behavior and varieties of the SARS-COV-2 virus are unknown, although it is true that the scientific-technical community has made a great effort in identifying the parameters which ensure the inactivity of the mentioned virus.

In this sense, research which has been conducted shown that the virus is thermolabile *in vitro.* In other words, the SARS-CoV-2 virus is highly stable at 4ºC when kept in a liquid medium. However, the concentration of the activity of this virus drops significantly as a function of increasing incubation temperature, verifying its inactivity in a duration of 7 days at a temperature of 22ºC, 1 day at 37ºC, 10 minutes at 56ºC, or 1 minute at a temperature of 70ºC.

Therefore, the SARS-CoV-2 virus is destroyed or inactivated by applying heat at a certain temperature for a specific time period as a result of the rupturing of its membrane or viral envelope.

The presence of the SARS-CoV-2 virus has forced the society to change its habits. Specifically, the need to develop an indicator textile material which allows ensuring the absence of coronavirus from clothing sold, from clothing returned to the establishment by customers and must be offered for sale again safely, or from clothing which consumers tried on but did not ultimately purchase, has been detected in the sales of textile clothing.

### DESCRIPTION OF THE INVENTION

The proposed biomarker solves the problems set forth above in a fully satisfactory manner, allowing the formation of a marker or textile indicator material impregnated with a suspension of artificial lipid particles which are similar to coronavirus in terms of size and morphology. Likewise, said lipid particles have an outer membrane containing a fat-soluble coloring agent.

Specifically, the biomarker is formed by a suspension of lipid particles formed by a plurality of spherical vesicles of a size between 100 nm and 1000 nm and wherein each spherical vesicle has an outer membrane made up of phospholipids.

Each phospholipid necessarily consists of a hydrophilic head and a hydrophobic tail, the hydrophilic head of the phospholipid being oriented towards the outside of the vesicle and the hydrophobic tail thereof being oriented towards the inside of the vesicle.

In this way, each vesicle contains therein a fat-soluble coloring agent which will be surrounded by the hydrophobic tails of the phospholipids making up the outer membrane of the vesicle.

Advantageously, the structure of the vesicles making up the biomarker of the invention behaves like a coronavirus, and to that end, the suspension of lipid particles allows the use thereof in generating an indicator suitable for identifying whether or not the impregnated textile material has been subjected to the necessary disinfection treatment to ensure the rupturing of the outer membrane or envelope of the suspended lipid particles.

More specifically, the developed biomarker offers means which allows identifying whether or not the textile material has been subjected to a disinfection treatment sufficient to ensure the absence of an active coronavirus. In this sense, the disinfection treatments applied to the textile material include, among others to be determined, dry heat, wet heat, ozone, and ultraviolets.

In this way, the textile indicator material is impregnated with the suspension of the invention and is stained by the fat-soluble coloring agent when the rupturing of the outer membranes of the vesicles making up the biomarker occurs. Therefore, the textile material changes to a color that can be readily detected by the human eye, which allows concluding that active coronavirus particles are absent in the textile material given that it has been subjected to a disinfection treatment sufficient to cause the rupturing of the envelope of the vesicles contained in the suspension of the invention. Therefore, the presence of the fat-soluble coloring agent in the textile indicator material confirms the inactivity of coronavirus.

The lipid particles making up the suspension of the invention or biomarker can therefore be solid lipid nanoparticles or liposomes.

When the lipid particles are solid lipid nanoparticles, the vesicles are made up of phospholipids which are ordered forming only a single lipid layer or row constituting the outer envelope of the vesicle.

However, when the lipid particles are liposomes, the vesicles are made up of phospholipids which organize into lipid bilayers constituting a double membrane which defines an intermediate space in which the fat-soluble coloring agent is contained. In this way, the hydrophobic tails of the phospholipids making up the lipid bilayers are oriented towards the intermediate space of the double membrane.

In any of the optional embodiments described above, the medium in which the lipid particles are suspended is preferably water.

The suspension of lipid particles has a concentration of between 10⁹ and 10¹⁴ particles per milliliter.

Standardized fabrics of different textile materials (100% cotton with EMPA 221 standard; 120 g/m² and multifiber indicator fabric according to ISO 105-F10 standard) are impregnated with the suspension of lipid particles, with a percentage of absorption of the biomarker solution of 65%, and air dried at room temperature.

Based on the foregoing, the object of the invention is to provide a biomarker, with a textile substrate being impregnated with said biomarker in order to constitute a marker or textile indicator material simulating the behavior of coronavirus for use thereof in the validation of the effectiveness of disinfection processes for clothing and different textile substrates, which are included in the field of household textiles, transports, etc.

The process for obtaining the suspension of lipid particles refers to closed entrapment or microencapsulation methods, in which the object is to obtain a phospholipid membrane which encloses or microencapsulates the fat-soluble coloring agent.

### BRIEF DESCRIPTION OF THE DRAWINGS

To complement the description that will be made below and for the purpose of helping to better understand the features of the invention according to a preferred practical embodiment thereof, a set of drawings is attached as an integral part of said description in which the following is depicted in an illustrative and non-limiting manner:
Figure 1 shows a view of the structure of a lipid particle of the invention formed by a solid lipid nanoparticle, as well as a partial reduction of the structure in which the outer membrane can be seen.
Figure 2 shows a view of the structure of a lipid particle of the invention formed by a liposome, as well as a partial reduction of the structure in which the outer membrane and the inner membrane can be seen.
Figure 3 shows the depiction of the intensity evolution of a textile indicator material, made according to a preferred embodiment of the biomarker of the invention, with respect to temperature and time, differentiating four different evolutions corresponding to four tested temperatures (A, B, C, and D).
Figure 4 shows the depiction of color intensity variation with a textile indicator material being steamed at 160ºC (line E) at a distance of one cm and with the textile indicator material being ironed at 160ºC (line F).
Figure 5 shows photomicrographs of the textile indicator materials impregnated with the biomarker of the invention which simulates coronavirus before thermal disinfection treatment.
Figure 6 shows photomicrographs of the textile indicator materials impregnated with the biomarker of the invention which simulates coronavirus after thermal disinfection treatment.

### PREFERRED EMBODIMENT OF THE INVENTION

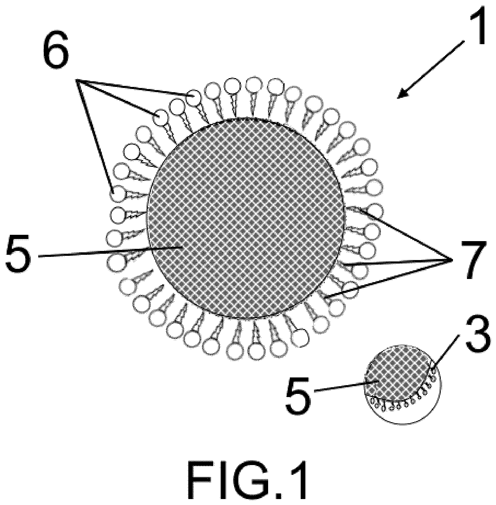

In a first preferred embodiment of the invention, the biomarker is made up of lipid particles which are solid lipid nanoparticles (1) and the phospholipids are ordered so as to form only a single lipid layer or row constituting the outer membrane (3) or outer envelope of the vesicle. The mentioned outer membrane (3) contains a fat-soluble coloring agent (5), as depicted in Figure 1.

The phospholipids of the outer membrane (3) are ordered such that their hydrophilic head (6) is oriented towards the outside of the vesicle and their hydrophobic tail (7) is oriented towards the inside of the vesicle.

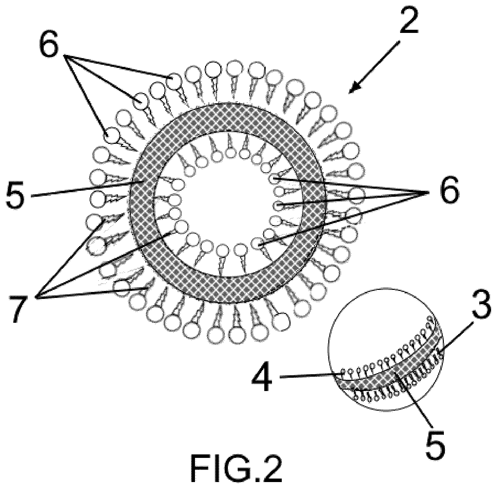

In a second preferred embodiment of the invention in which the biomarker is made up of lipid particles which are liposomes (2) such that the phospholipids are ordered so as to form liposomes. The vesicles are therefore made up of phospholipids which organize into lipid bilayers constituting a double membrane, i.e., an outer membrane (3) and an inner membrane (4) defining an intermediate space in which the fat-soluble coloring agent is contained (5). In this way, the hydrophobic tails (7) of the phospholipids making up the lipid bilayers are oriented towards the intermediate space between the outer membrane (3) and the inner membrane (4), as depicted in Figure 2.

In both embodiments of the invention, a preferably white textile indicator material is impregnated with the suspension, and the rupturing of the outer membrane (3) of the suspended lipid particles of the invention is also confirmed upon applying disinfection processes which theoretically destroy the virus.

In this way, the textile indicator material impregnated with the suspension of the invention will allow the release of the fat-soluble coloring agent enclosed in the outer membrane (3) thereof, giving color to the textile indicator material and indicating that the potential virus would also have been inactivated.

This section describes in detail the tests performed in order to verify the behavior of the biomarker formed by the suspension of lipid particles of the invention. Specifically, the behavior thereof with respect to thermal treatment is analyzed for a suspension according to the object of the invention in which the lipid particles are liposomes.

To perform the study, impregnation is carried out on 100% cotton fabrics (EMPA 221 standard; 120 g/m²) having a percentage of absorption of the suspension of liposomes of 65% and said textile materials are air dried at room temperature, thereby obtaining the textile indicator material impregnated with the biomarker of the invention.

The obtained textile indicator material is then subjected to one of the following thermal processes:
- Test 1: 60-minute exposure to the following temperatures in an oven: 60ºC, 65ºC, 75ºC, and 90ºC.
- Test 2: dry ironing at a temperature of 160ºC, applying different treatment times.
- Test 3: steaming with a steam cleaner at 160ºC, applying different treatment times and different distances with respect to the textile indicator material.

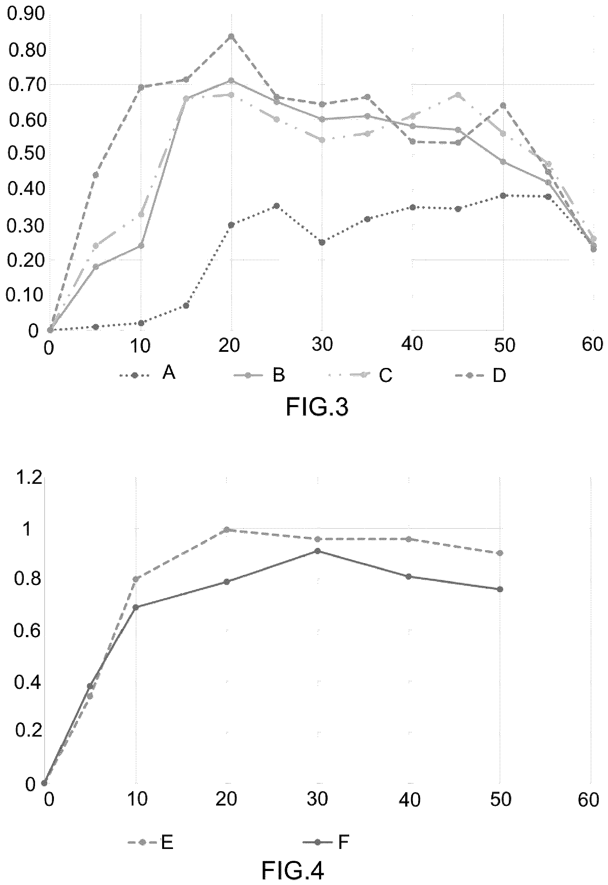

Figure 3 showing the measurement of color change as a result of the rupturing of the outer membrane (3) by means of colorimetry is prepared for test 1.

Specifically, Figure 3 shows the color intensity variation with respect to an untreated textile indicator material for four temperatures object of the study, namely, 60ºC, 65ºC, 75ºC, and 90ºC (linear representations A, B, C, and D).

In this way, Figure 3 depicts in the X-axis time in minutes and in the Y-axis the dimensionless measurement dL* which is defined as the difference in brightness or darkness measurement between the textile indicator material impregnated with the biomarker and which has undergone a thermal treatment analyzed with respect to a reference standard or indicator impregnated with the biomarker which has not been subjected to any disinfection treatment.

The textile indicator material is analyzed every 5 minutes in test 1.

The results of test 1 are shown in the following table:

| Time (min) | Temperature 60ºC (line A of Figure 3) | Temperature 65ºC (line B of Figure 3) | Temperature 75ºC (line C of Figure 3) | Temperature 90ºC (line D of Figure 3) |
|---|---|---|---|---|
| 0 | 0.00 | 0.00 | 0.00 | 0.00 |
| 5 | 0.01 | 0.18 | 0.24 | 0.44 |
| 10 | 0.02 | 0.24 | 0.33 | 0.69 |
| 15 | 0.07 | 0.66 | 0.66 | 0.71 |
| 20 | 0.30 | 0.71 | 0.67 | 0.84 |
| 25 | 0.35 | 0.65 | 0.60 | 0.66 |
| 30 | 0.25 | 0.60 | 0.54 | 0.64 |
| 35 | 0.32 | 0.61 | 0.56 | 0.66 |
| 40 | 0.35 | 0.58 | 0.61 | 0.54 |
| 45 | 0.34 | 0.57 | 0.67 | 0.53 |
| 50 | 0.38 | 0.48 | 0.56 | 0.64 |
| 55 | 0.38 | 0.42 | 0.47 | 0.45 |
| 60 | 0.24 | 0.24 | 0.26 | 0.23 |

The following conclusions can be made based on Figure 3 which shows the results of test 1:
- The trend and the behavior of the textile indicator material are repeated for the four tested temperatures (linear representations A, B, C, and D). However, as temperature increases, color change occurs sooner, i.e., the outer membrane of the liposome is destroyed and the color intensity in the textile indicator material increases, with all of them reaching the maximum after 20 minutes of exposure and remaining unchanged for the next 30 minutes.
- At a temperature of 60ºC (linear representation or line A), the change is very gradual and not as intense, which suggests that some liposomes could still be intact.
- At temperatures of 65ºC, 75ºC, and 90ºC (linear representations B, C, and D), the behavior is very similar. In other words, not only is a significant change in intensity observed after 5 minutes, but the color remains stable during the 50 minutes of treatment, after which the colorant is affected by prolonged exposure to temperature and degrades, losing intensity.

Next, the following table describes in detail the results obtained from tests 2 and 3 in which the textile indicator material impregnated with the biomarker of the invention is subjected to dry ironing at a temperature of 160ºC (test 2) applying different treatment times and to steaming at 160ºC (test 3) performed at a distance of one cm from the textile indicator material.

| Time (seconds) | Steaming (160ºC) (Line E of Figure 4) | Dry ironing (Line F of Figure 4) |
|---|---|---|
| 0 | 0 | 0 |
| 5 | 0.34 | 0.38 |
| 10 | 0.8 | 0.69 |
| 20 | 0.994 | 0.79 |
| 30 | 0.9575 | 0.91 |
| 40 | 0.9575 | 0.81 |
| 50 | 0.902 | 0.76 |

Likewise, Figure 4 shows:
- Color intensity variation with steaming at 160ºC (linear representation E) performed at a distance of one cm from the textile indicator material, and
- The color intensity variation with a dry ironing a 160ºC (linear representation F).

In this way, Figure 4 depicts in the X-axis exposure time in seconds of the textile indicator material and in the Y-axis the dimensionless measurement dL* which is defined as the difference in brightness or darkness measurement between the textile indicator material impregnated with the biomarker and which has undergone a thermal treatment analyzed with respect to a reference standard or indicator impregnated with the biomarker which has not been subjected to any disinfection treatment.

Again, the trend, and therefore, the behavior of the textile indicator material impregnated with a biomarker of the present invention, are similar to the foregoing, but in test 2 it is more similar to the behavior of the textile indicator material subjected to 90ºC (representation D of Figure 3) in the oven, given that the change in color intensity occurs with 10 seconds of exposure, with the maximum being reached after 20 seconds of exposure of the textile indicator material.

The following table shows the temperatures reached on the surface of the textile indicator material based on the distance at which steaming is applied (test 3):

| Distance (cm) | Temperature (ºC) |
|---|---|
| 1 | 79 |
| 5 | 68 |
| 10 | 58 |

On the other hand, the following table shows the temperatures reached on the surface of the textile indicator material based on the time in seconds the ironing is applied (test 2):

| Distance (cm) | Temperature (ºC) |
|---|---|
| 5 | 101 |
| 10 | 125 |
| 15 | 130 |

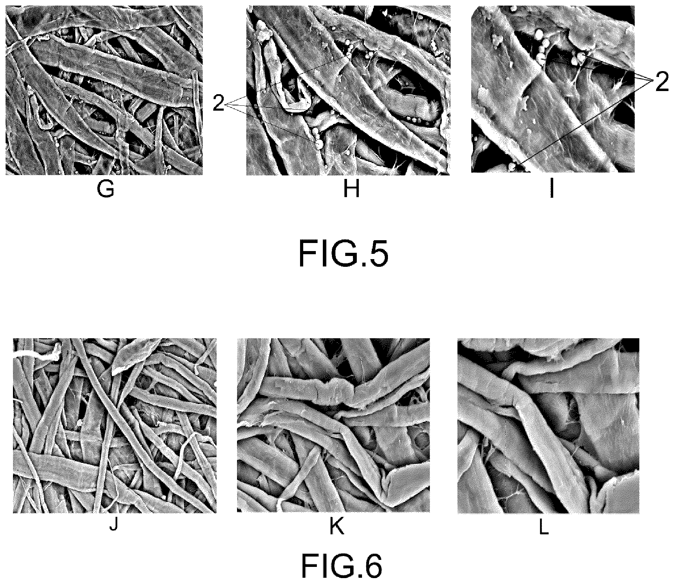

Finally, the tested textile indicator materials impregnated with a biomarker according to the present invention are observed by means of SEM microscopy before (Figure 5) and after (Figure 6) being subjected to thermal treatments. Specifically, Figures 5 and 6 correspond to electron microscopy images, respectively.

Figure 5 shows photomicrographs of the cotton textile indicator materials impregnated with the suspension of the biomarker which simulates coronavirus before thermal treatment. Specifically, the photomicrographs are reproduced without applying any magnification (Figure 5, letter G), with 50x magnification (Figure 5, letter H), and with 100x magnification (Figure 5, letter I).

Figure 6 shows photomicrographs of the cotton textile indicator materials impregnated with the suspension of the biomarker which simulates coronavirus after thermal treatment. Specifically, the photomicrographs are reproduced without any amplification (Figure 6, letter J), with 50x amplification (Figure 6, letter K), and with 100x amplification (Figure 6, letter L).

Figure 5 shows the liposomes distributed throughout the textile indicator material before thermal treatment, whereas Figure 6 shows the textile indicator material after being subjected to thermal treatment. As can be seen in the photographs of Figures 5 and 6, the liposomes (2) present in Figure 5 have completely disappeared in Figure 6, not leaving any trace after thermal treatment.

It can therefore be concluded that the disappearance of the outer membrane of the vesicles making up the suspended particles takes place with thermal treatment applied, simulating a behavior similar to coronavirus particles.

## Claims

1. A biomarker formed by a suspension of lipid particles formed by a plurality of spherical vesicles of a size between 100 nm and 1000 nm, wherein each spherical vesicle has an outer membrane made up of phospholipids and each phospholipid consists of a hydrophilic head and a hydrophobic tail, the hydrophilic head of the phospholipid being oriented towards the outside of the vesicle and the hydrophobic tail thereof being oriented towards the inside of the vesicle, and wherein each vesicle contains a fat-soluble coloring agent (non-water-soluble) for use thereof in the validation of the effectiveness of thermal disinfection processes against coronavirus-type virus.

2. The biomarker for use thereof in the validation of the effectiveness of thermal disinfection processes against coronavirus-type virus according to claim 1, **characterized in that** the lipid particles are solid lipid nanoparticles.

3. The biomarker for use thereof in the validation of the effectiveness of thermal disinfection processes against coronavirus-type virus according to claim 1, **characterized in that** the lipid particles are liposomes.

4. The biomarker for use thereof in the validation of the effectiveness of thermal disinfection processes against coronavirus-type virus according to claim 3, **characterized in that** the phospholipids are organized in lipid bilayers constituting a double membrane which defines an intermediate space in which the fat-soluble coloring agent is contained, the hydrophobic tails of the phospholipids making up the lipid bilayers being oriented towards the intermediate space of the double membrane.

5. The biomarker for use thereof in the validation of the effectiveness of thermal disinfection processes against coronavirus-type virus according to any of the preceding claims, **characterized in that** the medium in which the lipid particles are suspended is water.

6. The biomarker for use thereof in the validation of the effectiveness of thermal disinfection processes against coronavirus-type virus according to any of the preceding claims, **characterized in that** a textile substrate is impregnated with same.

7. A validation method for validating the effectiveness of thermal disinfection processes against coronavirus-type virus according to any of the preceding claims, comprising the steps of:
- impregnating a textile substrate with the biomarker,
- applying thermal disinfection processes,
- rupturing the outer membrane made up of phospholipids of the spherical vesicles of the suspended lipid particles of the biomarker,
- releasing the coloring agent contained in the spherical vesicles forming the suspended lipid particles of the biomarker,
- coloring the textile substrate impregnated with the biomarker,
- identifying, by the human eye, the rupturing of the outer membrane of the spherical vesicles forming the suspended lipid particles of the biomarker indicating that the potential virus has been inactivated, which represents the destruction/absence of the virus contained in the textile material.

8. The validation method for validating the effectiveness of thermal disinfection processes against coronavirus-type virus according to claim 7, **characterized in that** the thermal disinfection process is selected from dry heat, wet heat, ozone, and ultraviolets.

## Amended claims

### Amended claims under Art. 19.1 PCT

1. A validation method for validating the effectiveness of thermal disinfection processes against coronavirus-type virus, comprising the steps of:
- impregnating a textile substrate with a biomarker, wherein the biomarker is formed by a suspension of lipid particles formed by a plurality of spherical vesicles of a size between 100 nm and 1000 nm, wherein each spherical vesicle has an outer membrane made up of phospholipids and each phospholipid consists of a hydrophilic head and a hydrophobic tail, the hydrophilic head of the phospholipid being oriented towards the outside of the vesicle and the hydrophobic tail thereof being oriented towards the inside of the vesicle, and wherein each vesicle contains a fat-soluble coloring agent (non-water-soluble),
- applying thermal disinfection processes,
- rupturing the outer membrane made up of phospholipids of the spherical vesicles of the suspended lipid particles of the biomarker,
- releasing the coloring agent contained in the spherical vesicles forming the suspended lipid particles of the biomarker,
- coloring the textile substrate impregnated with the biomarker,
- identifying, by the human eye, the rupturing of the outer membrane of the spherical vesicles forming the suspended lipid particles of the biomarker indicating that the potential virus has been inactivated, which represents the destruction/absence of the virus contained in the textile material.

2. The validation method for validating the effectiveness of thermal disinfection processes against coronavirus-type virus according to claim 1, **characterized in that** the thermal disinfection process is selected from dry heat, wet heat, ozone, and ultraviolets.
